(19) Europäisches Patentamt
European Patent Office
Office européen des brevets

(11) **EP 3 191 190 B1**

(12) **EUROPÄISCHE PATENTSCHRIFT**

(45) Veröffentlichungstag und Bekanntmachung des Hinweises auf die Patenterteilung:
**15.01.2020 Patentblatt 2020/03**

(21) Anmeldenummer: **15753015.5**

(22) Anmeldetag: **18.08.2015**

(51) Int Cl.:
***A61Q 5/10*** (2006.01) ***A61K 8/898*** (2006.01)
***A61Q 5/06*** (2006.01)

(86) Internationale Anmeldenummer:
**PCT/EP2015/068877**

(87) Internationale Veröffentlichungsnummer:
**WO 2016/037804 (17.03.2016 Gazette 2016/11)**

(54) **OXIDATIVE AUFHELLMITTEL MIT SPEZIELLEN AMINIERTEN SILICONPOLYMEREN**

OXIDATIVE HAIR LIGHTENERS WITH SPECIAL AMINATED SILCONE POLYMERS

COMPOSITION D'ÉCLAIRCISSEMENT OXYDANTE COMPORTANT DES POLYMÈRES DE SILICONE AMINÉS SPÉCIAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.09.2014 DE 102014217999**

(43) Veröffentlichungstag der Anmeldung:
**19.07.2017 Patentblatt 2017/29**

(73) Patentinhaber: **Henkel AG & Co. KGaA**
**40589 Düsseldorf (DE)**

(72) Erfinder:
- **KERL, Sylvia**
  **22763 Hamburg (DE)**
- **BIETZ, Susanne**
  **25336 Elmshorn (DE)**

(56) Entgegenhaltungen:

| | |
|---|---|
| **EP-A1- 1 464 321** | **WO-A2-02/47632** |
| **WO-A2-03/009822** | **WO-A2-2012/079915** |
| **FR-A1- 2 895 242** | **US-A1- 2003 152 534** |
| **US-A1- 2003 229 947** | **US-A1- 2007 261 178** |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 191 190 B1

Printed by Jouve, 75001 PARIS (FR)

## Beschreibung

Die vorliegende Erfindung betrifft kosmetische Mittel zur Färbung, insbesondere Aufhellung, keratinischer Fasern, welche spezielle aminierte Siliconpolymere enthalten.

Weiterhin betrifft die vorliegende Erfindung eine Verpackungseinheit (Kit-of-parts), enthaltend ein erfindungsgemäßes kosmetisches Mittel sowie eine Oxidationsmittelzubereitung.

Darüber hinaus betrifft die vorliegende Erfindung ein Verfahren zum Färben, insbesondere Aufhellen, von keratinischen Fasern unter Verwendung eines erfindungsgemäßen kosmetischen Mittels sowie einer Oxidationsmittelzubereitung.

Zudem betrifft die vorliegende Erfindung die Verwendung eines erfindungsgemäßen kosmetischen Mittels zur Erhöhung der Pflege keratinischer Fasern bei gleichzeitig verbesserter Aufhellleistung.

Schließlich betrifft die vorliegende Erfindung die Verwendung einer erfindungsgemäßen Verpackungseinheit zur Herstellung eines kosmetischen Mittels zur Farbveränderung, insbesondere Aufhellung, keratinischer Fasern mit erhöhter Pflege der keratinischen Fasern bei gleichzeitig verbesserter Aufhellleistung.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln. Diese Mittel sollen neben einer hohen Färbeleistung zusätzliche Eigenschaften, wie beispielsweise die Steigerung des Haarvolumens, aufweisen.

Zur Bereitstellung farbverändernder kosmetischer Mittel, insbesondere für die Haut oder für keratinhaltige Fasern, wie beispielsweise menschliche Haare, sind im Stand der Technik verschiedene Färbesysteme bekannt.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss jedoch üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als oxidativen Färbungen, so dass sehr viel früher eine vielfach unerwünschte Nuancenverschiebung oder ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Insbesondere bei mehrfacher Anwendung von Mitteln mit 5,6-Dihydroxyindolin ist es möglich, bei Personen mit ergrauten Haaren die natürliche Haarfarbe wiederherzustellen. Die Ausfärbung kann dabei mit Luftsauerstoff als einzigem Oxidationsmittel erfolgen, so dass auf weitere Oxidationsmittel verzichtet werden kann. Bei Personen mit ursprünglich mittelblondem bis braunem Haar kann das 5,6-Dihydroxyindolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Im Stand der Technik werden Verpackungseinheiten zur Färbung keratinischer Fasern offenbart, welche getrennt konfektioniert a) ein kosmetisches Mittel, welches Oxidationsfarbstoffvorprodukte und/oder direktziehende Farbstoffe und ein Silikon sowie b) eine Oxidationsmittelzubereitung, enthaltend 0,5 bis 15 Gew.-% Oxidationsmittel enthalten. Beispielhaft sind hier die US 2003/152534 A1, die WO 03/009822 A2, die US 2003/229947 A1, die WO 02/47632 A2, die US 2007/261178 A1, die EP 1 464 321 A1, die FR 2 895 242 A1 und die WO 2012/079915 A2 zu nennen.

Die im Stand der Technik bekannten Färbemittel führen jedoch nicht immer zu der gewünschten hohen Färbeleistung, insbesondere Aufhellleistung, oder weisen zusätzliche gewünschte Eigenschaften, wie beispielsweise eine verbesserte Pflege der Haare während der Haarfärbung, auf.

Der vorliegenden Erfindung lag daher die Aufgabe zugrunde, ein kosmetisches Mittel zur Färbung, insbesondere Aufhellung, keratinischer Fasern bereitzustellen, welches die Nachteile des Standes der Technik vermeidet bzw. zumindest abschwächt und welches in einer verbesserten Pflege der Haare bei gleichzeitig verbesserter Aufhellleistung resultiert.

Es wurde nun überraschenderweise gefunden, dass der Zusatz von mindestens einem speziellen aminierten Siliconpolymer in kosmetischen Mitteln zur Färbung, insbesondere Aufhellung, keratinischer Fasern, insbesondere menschlicher Haare, zu einer verbesserten Pflege, insbesondere zu einer verbesserten Kämmbarkeit, bei gleichzeitig verbesserter Aufhellleistung führt.

Ein erster Gegenstand der Erfindung ist daher ein kosmetisches Mittel zur Farbveränderung, insbesondere Aufhellung, keratinischer Fasern, umfassend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,
b) mindestens ein aminiertes Siliconpolymer, enthaltend mindestens eine Struktureinheit der Formel (I) und mindestens eine Struktureinheit der Formel (II)

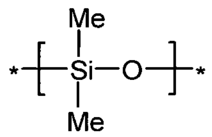
(I)

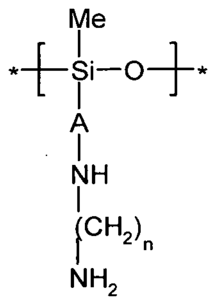
(II),

worin

A für eine lineare oder verzweigte $C_4$-$C_8$-Alkylgruppe steht, und
n für ganze Zahlen von 1 bis 4 steht,

wobei

- das kosmetische Mittel keine Säure(n), ausgewählt aus der Gruppe der Carbonsäuren mit 8 bis 30 Kohlenstoffatomen, der Ethercarbonsäuren mit 8 bis 30 Kohlenstoffatomen, der Etherphosphorsäuren mit 8 bis 30 Kohlenstoffatomen, der Phosphorsäuren mit 8 bis 30 Kohlenstoffatomen sowie deren Mischungen, enthält;
- das kosmetische Mittel mindestens ein aminiertes Siliconpolymer der Formel (III) enthält

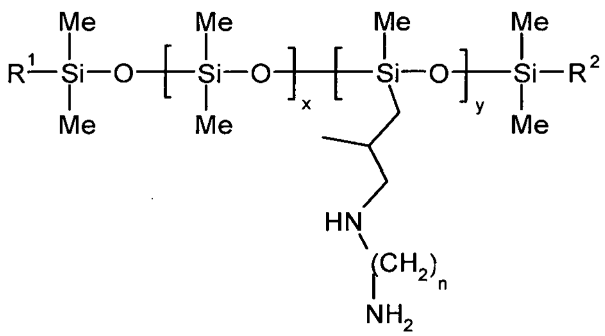
(III),

worin

$R^1$ und $R^2$, jeweils unabhängig voneinander, für eine Methylgruppe oder eine Hydroxylgruppe stehen,
x für ganze Zahlen von 49 bis 149, steht,
y für ganze Zahlen von 1 bis 10, steht, und
n für 2 oder 3, steht

- das kosmetische Mittel zusätzlich ein cyclisches aminiertes Siliconpolymer in einer Gesamtmenge von 0,015 bis 0,3 Gew.-%, bezogen auf das Gesamtgewicht des kosmetischen Mittels, enthält, wobei das cyclische aminierte Siliconpolymer die Formel (IV) aufweist

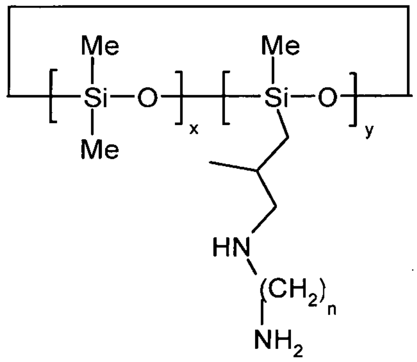

(IV),

worin

x für ganze Zahlen von 49 bis 149, steht, und
y für ganze Zahlen von 1 bis 10, steht.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Färbung von menschlichen Haaren verwendet werden.

Weiterhin werden unter dem Begriff "aminierte Siliconpolymere" im Rahmen der vorliegenden Erfindung Siliconpolymere verstanden, welche mindestens eine Aminogruppe pro Siliconpolymer aufweisen.

Zudem wird unter dem Begriff "Kämmbarkeit" im Rahmen der vorliegenden Erfindung sowohl die Kämmbarkeit der nassen Faser, als auch die Kämmbarkeit der trockenen Faser verstanden.

Erfindungsgemäß fallen unter Carbonsäuren mit 8 bis 30 Kohlenstoffatomen Carbonsäuren der Formel R-COOH, worin R für einen linearen oder verzweigten, gesättigten oder ungesättigten Alkylrest mit 8 bis 30 Kohlenstoffatomen steht. Beispiele für derartige Carbonsäuren sind beispielsweise Caprinsäure, Laurinsäure, Kokosnusssäure, Stearinsäure, Isostearinsäure, Linolensäure, Ölsäure, Myristinsäure, Olivenölsäure und dergleichen. Unter die Gruppe der Ethercarbonsäuren mit 8 bis 30 Kohlenstoffatomen fallen erfindungsgemäß Verbindungen der Formel $RO[CH_2O]_u[(CH_2)_xCH(R')(CH_2)_y(CH_2)_zO]_v[CH_2CH_2O]_wCH_2COOH$, worin R für einen linearen oder verzweigten, gesättigten oder ungesättigten Alkylrest mit 8 bis 30 Kohlenstoffatomen steht, R' für eine Hydroxylgruppe oder eine Alkylgruppe steht, u, v und w, jeweils unabhängig voneinander, für ganze Zahlen von 0 bis 60 stehen und x, y und y, jeweils unabhängig voneinander für ganze Zahlen von 0 bis 13 stehen, wobei die Summe $x+y+z \geq 0$ beträgt. Beispiele für derartige Ethercarbonsäuren sind beispielsweise die unter den INCI-Bezeichnungen bekannten Verbindungen Laureth-4 Carboxylic Acid, PPG-6-Laureth-6 Carboxylic Acid, Steareth-7 Carboxylic Acid, Oleth-10 Carboxylic Acid, Trideceth-6 Carboxylic Acid, Undeceth-5 Carboxylic Acid und dergleichen. Etherphosphorsäuren mit 8 bis 30 Kohlenstoffatomen im Rahmen der vorliegenden Erfindung fallen unter die Formel $\{RO[CH_2O]_u[(CH_2)_xCH(R')(CH_2)_y(CH_2)_zO]_v[CH_2CH_2O]_w\}_a\text{-PO-(OH)}_b$, worin R für einen linearen oder verzweigten, gesättigten oder ungesättigten Alkylrest mit 8 bis 30 Kohlenstoffatomen steht, R' für eine Hydroxylgruppe oder eine Alkylgruppe steht, u, v und w, jeweils unabhängig voneinander, für ganze Zahlen von 0 bis 60 stehen, x, y und y, jeweils unabhängig voneinander für ganze Zahlen von 0 bis 13 stehen, b für die ganze Zahl 0 steht wenn a für die ganze Zahl 2 steht und b für die ganze Zahl 2 steht wenn a für die ganze Zahl 0 steht, wobei die Summe $x+y+z \geq 0$ beträgt. Beispiele für derartige Etherphosphorsäuren sind beispielsweise PPG-5-Ceteth-10 phosphat, Oleth-3 phosphat und dergleichen. Schließlich fallen unter die Gruppe der Phosphorsäuren mit 8 bis 30 Kohlenstoffatomen erfindungsgemäß Verbindungen der Formel $R\text{-O-P(O)(OH)}_2$, worin R für einen linearen oder verzweigten, gesättigten oder ungesättigten Alkylrest mit 8 bis 30 Kohlenstoffatomen steht. Beispiele für derartige Phosphorsäuren sind beispielsweise Caprylphosphat, Laurylphosphat, Oleylphosphat, Isostearylphosphat, Stearylphosphat und Cetylphosphat.

Darüber hinaus werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen Mittels bezieht sich vorliegend - sofern nichts anderes angegeben ist - auf die Gesamtmenge an Aktivsubstanz der jeweiligen Komponente. Weiterhin bezieht sich die Angabe der Gesamtmenge in Bezug auf die Komponenten des kosmetischen Mittels - sofern nichts anderes angegeben ist - auf das Gesamtgewicht des oxidationsmittelfreien erfindungsgemäßen kosmetischen Mittels.

Die erfindungsgemäßen kosmetischen Mittel enthalten einen kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger enthält im Sinne der Erfindung enthält mindestens 30 Gew.-%, insbesondere mindestens 50 Gew.-%, Wasser, bezogen auf das Gesamtgewicht des kosmetischen Mittels.

Unter wässrig-alkoholischen Trägern sind im Sinne der vorliegenden Erfindung wasserhaltige Zusammensetzungen, enthaltend einen $C_1$-$C_4$-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Das erfindungsgemäße kosmetische Mittel enthält als ersten wesentlichen Bestandteil a) eine Verbindung, ausgewählt aus der Gruppe von Oxidationsfarbstoffvorprodukten (OFV), direktziehenden Farbstoffe (DZ) sowie deren Mischungen.

In einer bevorzugten Ausführungsform enthalten erfindungsgemäße kosmetische Mittel mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige Verbindungen im erfindungsgemäßen kosmetischen Mittel enthalten sein. In einer bevorzugten Ausführungsform enthalten die erfindungsgemäßen kosmetischen Mittel daher mindestens ein Oxidationsfarbstoffvorprodukt vom Entwicklertyp. Es kann im Rahmen der vorliegenden Erfindung jedoch auch vorgesehen sein, dass die erfindungsgemäßen kosmetischen Mittel mindestens ein Oxidationsfarbstoffvorprodukt vom Kupplertyp enthalten. Besonders gute Ergebnisse in Bezug auf die Färbung keratinischer Fasern werden erhalten, wenn die erfindungsgemäßen kosmetischen Mittel mindestens ein Oxidationsfarbstoffvorprodukt vom Entwicklertyp und mindestens ein Oxidationsfarbstoffvorprodukt vom Kupplertyp enthalten.

Die Entwickler- und Kupplerkomponenten werden üblicherweise in freier Form eingesetzt. Bei Substanzen mit Aminogruppen kann es jedoch bevorzugt sein, deren Salzform, insbesondere in Form der Hydrochloride und Hydrobromide oder der Sulfate, einzusetzen.

Erfindungsgemäß sind kosmetische Mittel bevorzugt, welche die Entwickler- und/oder Kupplerkomponenten jeweils in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthalten.

In einer weiteren bevorzugten Ausführungsform ist das erfindungsgemäße kosmetische Mittel daher dadurch gekennzeichnet, dass es ein Oxidationsfarbstoffvorprodukt vom Entwickler und/oder Kupplertyp in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthält.

Geeignete Oxidationsfarbstoffvorprodukte vom Entwicklertyp sind beispielsweise p-Phenylendiamin und dessen Derivate. Bevorzugte p-Phenylendiamine werden ausgewählt aus einer oder mehrerer Verbindungen der Gruppe, welche gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, welche mindestens zwei aromatische Kerne enthalten, die mit Amino- und/oder Hydroxylgruppen substituiert sind. Bevorzugte zweikernige Entwicklerkomponenten werden ausgewählt aus N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan, Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol, 4-Amino-2-(diethylaminomethyl)phenol sowie deren physiologisch verträglichen Salze.

Ferner kann die Entwicklerkomponente ausgewählt sein aus o-Aminophenol und dessen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind 2,4,5,6-Tetraaminopyrimidin, 4-Hydroxy-2,5,6-triaminopyrimidin und deren physiologisch verträgliche Salze. Ein bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, welche gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen.

Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Gemäß einer weiteren bevorzugten Ausführungsform der vorliegenden Erfindung enthält das erfindungsgemäße kosmetische Mittel als Oxidationsfarbstoffvorprodukt neben mindestens einer Entwicklerkomponente weiterhin zusätzlich mindestens eine Kupplerkomponente. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus

(A) m-Aminophenol und dessen Derivaten, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivaten, wie 2-Amino-5-ethylphenol,
(C) m-Diaminobenzol und dessen Derivaten, wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,
(D) o-Diaminobenzol und dessen Derivaten,
(E) Di- beziehungsweise Trihydroxybenzolderivaten, insbesondere Resorcin, 2-Chlorresorcin, 4-Chlorresorcin, 2-Methylresorcin und 1,2,4-Trihydroxybenzol,
(F) Pyridinderivaten, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxy-pyridin,
(G) Naphthalinderivaten, wie 1-Naphthol und 2-Methyl-1-naphthol,
(H) Morpholinderivaten, wie 6-Hydroxybenzomorpholin,
(I) Chinoxalinderivaten,
(J) Pyrazolderivaten, wie 1-Phenyl-3-methylpyrazol-5-on,
(K) Indolderivaten, wie 6-Hydroxyindol,
(L) Pyrimidinderivaten oder
(M) Methylendioxybenzolderivaten, wie 1-(2'-Hydroxyethyl)-amino-3,4-methylendioxybenzol sowie deren physiologisch verträglichen Salze.

Erfindungsgemäß bevorzugte Kupplerkomponenten sind ausgewählt aus der Gruppe, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-di-aminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methyl-

phenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 4-Chlorresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie deren physiologisch verträgliche Salze.

In einer besonders bevorzugten Ausführungsform der vorliegenden Erfindung sind die erfindungsgemäßen kosmetischen Mittel dadurch gekennzeichnet, dass sie als Oxidationsfarbstoffvorprodukt mindestens eine Entwicklerkomponente, ausgewählt aus der Gruppe von p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol, deren physiologisch verträglichen Salze sowie deren Mischungen, und mindestens eine Kupplerkomponente, ausgewählt aus der Gruppe von Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin, deren physiologisch verträglichen Salze sowie deren Mischungen, enthalten.

Um eine ausgewogene und subtile Nuancenausbildung zu erhalten, kann es im Rahmen der vorliegenden Erfindung auch vorgesehen sein, dass die erfindungsgemäßen kosmetischen Mittel zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, welche mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das erfindungsgemäße kosmetische Mittel die direktziehenden Farbstoffe in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels.

Als zweiten wesentlichen Bestandteil b) enthalten die erfindungsgemäßen kosmetischen Mittel mindestens ein spezielles aminiertes Siliconpolymer. Der Zusatz dieser speziellen Siliconpolymere führt zu einer verbesserten Pflege, insbesondere verbesserten Nasskämmbarkeit, bei gleichzeitig verbesserter Aufhellleistung der erfindungsgemäßen kosmetischen Mittel.

Gemäß einer bevorzugten Ausführungsform der vorliegenden Erfindung steht in der Struktureinheit der Formel (II) n für die ganze Zahl 2 oder 3, insbesondere für die ganze Zahl 2, und A für eine verzweigte $C_4$-Alkylgruppe, insbesondere eine Isobutylgruppe.

Erfindungsgemäß enthält das kosmetische Mittel mindestens ein aminiertes Siliconpolymer der Formel (III)

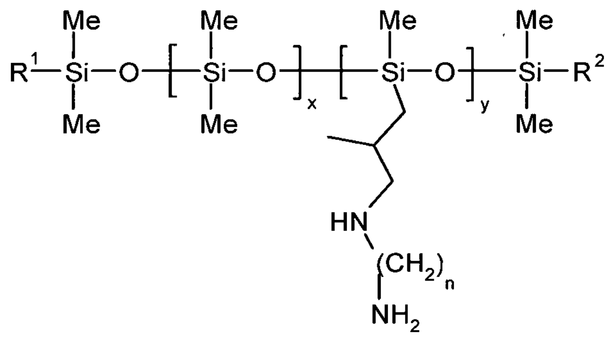

(III),

worin

$R^1$ und $R^2$, jeweils unabhängig voneinander, für eine Methylgruppe oder eine Hydroxylgruppe stehen,
x für ganze Zahlen von 49 bis 149, steht,
y für ganze Zahlen von 1 bis 10, steht, und
n für 2 oder 3, steht.

Der Einsatz dieser speziellen aminierten Siliconpolymere resultiert in einer erhöhten Pflege der keratinischen Fasern, insbesondere erhöhten Nasskämmbarkeit, nach der Farbveränderung, insbesondere Aufhellung, und führt gleichzeitig zu einer verbesserten Aufhellung.

Bevorzugt weist das mindestens eine aminierte Siliconpolymer b) ein mittleres Molekulargewicht $M_w$ von 350 bis 350.000 Da, vorzugsweise von 500 bis 300.000 Da, bevorzugt von 700 bis 250.000 Da, insbesondere von 1.000 bis 200.000 Da, auf. Spezielle aminierte Siliconpolymere, welche das zuvor angeführte mittlere Molekulargewicht $M_w$ aufweisen, resultieren in einer besonders hohen Pflege keratinischer Fasern nach der Farbveränderung bei gleichzeitig verbesserter Aufhellleistung. Das mittlere Molekulargewicht $M_w$ kann beispielsweise durch Gelpermeationschromatographie (GPC) bestimmt werden (Liu X. M. et. al.; "Comparative Studies of Poly(DimethylSiloxanes) Using Automated GPC-MALDI-TOF MS and On-Line GPC-ESI-TOF MS"; Am. Soc. Mass. Spectrom., 2003, 14, Seiten 195 bis 202).

Es hat sich als vorteilhaft erwiesen, wenn das mindestens eine aminierte Siliconpolymer b) eine Aminzahl oberhalb von 0,25 meq/g, vorzugsweise oberhalb von 0,3 meq/g, insbesondere oberhalb von 0,4 meq/g, aufweist.

Besonders bevorzugt ist es im Rahmen der vorliegenden Erfindung, wenn das mindestens eine aminierte Siliconpolymer b) eine Aminzahl von 0,25 bis 5 meq/g, vorzugsweise von 0,3 bis 4,5 meq/g, bevorzugt von 0,4 bis 4,0 meq/g, weiter bevorzugt von 0,5 bis 3,0 meq/g, insbesondere von 0,5 bis 1,5 meq/g, aufweist. Die Aminzahl steht dabei für die Milli-Äquivalente Amin pro Gramm des aminofunktionellen Silikons. Sie kann durch Titration ermittelt und auch in der Einheit mg KOH/g angegeben werden. Der Einsatz von aminierten Siliconpolymeren mit den zuvor genannten Aminzahlen resultiert in einer besonders hohen Pflege der mit den erfindungsgemäßen kosmetischen Mitteln behandelten keratinischen Fasern. Weiterhin wird bei Einsatz von Siliconpoylmeren mit Aminzahlen in den zuvor genannten Bereichen eine hervorragende Aufhellleistung der erfindungsgemäßen kosmetischen Mittel erreicht.

Das mindestens eine aminierte Siliconpolymer b) ist in den erfindungsgemäßen kosmetischen Mitteln in einer Gesamtmenge von 0,0001 bis 15 Gew.-%, vorzugsweise von 0,0005 bis 10 Gew.-%, bevorzugt von 0,005 bis 5,0 Gew.-%, weiter bevorzugt von 0,01 bis 3,0 Gew.-%, insbesondere von 0,05 bis 1,0 Gew.-%, bezogen auf das Gesamtgewicht des kosmetischen Mittels, enthalten. Der Einsatz der zuvor genannten Gesamtmenge des speziellen aminierten Siliconpolymers führt zu einer erhöhten Pflege der keratinischen Fasern bei gleichzeitig hervorragender Aufhellleistung.

Es hat sich gezeigt, dass ein Zusatz an cyclischen aminierten Siliconpolymeren im Rahmen der vorliegenden Erfindung besonders vorteilhaft ist. Erfindungsgemäße kosmetische Mittel enthalten daher zusätzlich ein cyclisches aminiertes Siliconpolymer in einer Gesamtmenge von 0,015 bis 0,3 Gew.-%, bezogen auf das Gesamtgewicht des kosmetischen Mittels, wobei das cyclische aminierte Siliconpolymer die Formel (IV) aufweist

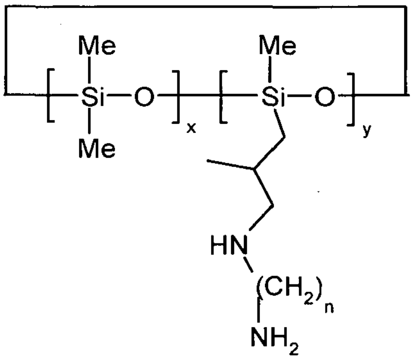

(IV),

worin

x für ganze Zahlen von 49 bis 149, steht, und
y für ganze Zahlen von 1 bis 10, steht.

Durch den Zusatz von aminierten cyclischen Siliconpolymeren kann die Pflegewirkung sowie die Aufhellleistung der erfindungsgemäßen kosmetischen Mittel weitergehend gesteigert werden.

Weiterhin hat sich der Zusatz von Dimethylcyclosiloxanen als vorteilhaft erweisen. Es ist daher im Rahmen der vorliegenden Erfindung bevorzugt, wenn die erfindungsgemäßen kosmetischen Mittel zusätzlich Dimethylcyclosiloxan in einer Gesamtmenge von weniger als 1 Gew.-%, bezogen auf das Gesamtgewicht des kosmetischen Mittels, enthalten, wobei das Dimethylcyclosiloxan die Formel (V) aufweist

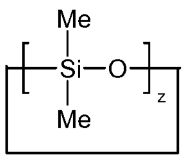

(V),

worin
z für ganze Zahlen von 2 bis 8, vorzugsweise von 2 bis 6, insbesondere für 3, 4, 5 oder 6, steht. Der Zusatz derartiger Dimethylcyclosiloxane zu den erfindungsgemäßen kosmetischen Mitteln führt zu einer weitergehenden Verbesserung der Pflegeleistung und der Aufhellleistung der erfindungsgemäßen kosmetischen Mittel.

Die erfindungsgemäßen kosmetischen Mittel können weitere Wirk- und Zusatzstoff enthalten. Es ist daher im Rahmen der vorliegenden Erfindung bevorzugt, wenn das kosmetische Mittel zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

Bevorzugt werden die erfindungsgemäßen kosmetischen Mittel als fließfähige Zubereitungen formuliert. Dabei sollten die kosmetischen Mittel so formuliert werden, dass diese sich einerseits gut am Anwendungsort auftragen und verteilen lassen, andererseits jedoch ausreichend viskos sind, so dass sie während der Einwirkzeit am Wirkort verbleiben und nicht verlaufen.

Es hat sich daher erfindungsgemäß als vorteilhaft erwiesen, wenn die erfindungsgemäßen kosmetischen Mittel mindestens ein Verdickungsmittel aus der Gruppe von (i) anionischen, synthetischen Polymeren; (ii) kationischen, synthetischen Polymeren; (iii) natürlich vorkommenden Verdickungsmitteln, wie nichtionischen Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektinen, Alginaten, Stärke-Fraktionen und Derivaten, wie Amylose, Amylopektin und Dextrinen, sowie Cellulosederivaten, wie beispielsweise Methylcellulose, Carboxyalkylcellulosen und Hydroxyalkylcellulosen; (iv) nichtionischen, synthetischen Polymeren, wie Polyvinylalkohol oder Polyvinylpyrrolidinon; (v) anorganischen Verdickungsmitteln, insbesondere Schichtsilikaten wie beispielsweise Bentonit, besonders Smektite, wie Montmorillonit oder Hectorit; sowie (vi) deren Mischungen, in einer Gesamtmenge von 0,0005 bis 5,0 Gew.-%, vorzugsweise von 0,001 bis 3,0 Gew.-%, bevorzugt von 0,005 bis 1,0 Gew.-%, insbesondere von 0,008 bis 0,01 Gew.-%, bezogen auf das Gesamtgewicht des kosmetischen Mittels, enthalten.

Es hat sich im Rahmen der vorliegenden Erfindung als besonders vorteilhaft erwiesen, wenn als Verdickungsmittel mindestens ein natürlich vorkommendes Verdickungsmittel, insbesondere Xanthangum sowie dessen Salze, in

einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,1 Gew.-%, bezogen auf das Gesamtgewicht des kosmetischen Mittels, enthalten ist.

Im Rahmen der vorliegenden Erfindung kann es bevorzugt sein, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 1,0 bis 35 Gew.-%, vorzugsweise von 5,0 bis 30 Gew.-%, bevorzugt von 10 bis 25 Gew.-%, insbesondere von 12 bis 20 Gew.-%, bezogen auf das Gesamtgewicht des kosmetischen Mittels, enthalten ist.

Bevorzugt können die erfindungsgemäßen kosmetischen Mittel weiterhin mindestens einen Partialester aus einem Polyol mit 2 bis 6 Kohlenstoffatomen und linearen gesättigten Carbonsäuren mit 12 bis 30, insbesondere 14 bis 22 Kohlenstoffatomen, wobei die Partialester hydroxyliert sein können, in einer Gesamtmenge von 0,5 bis 10 Gew.-%, insbesondere von 3,0 bis 8,0 Gew.-%, bezogen auf das Gesamtgewicht des kosmetischen Mittels, enthalten. Solche Partialester sind insbesondere die Mono- und Diester von Glycerin oder die Monoester von Propylenglycol oder die Mono- und Diester von Ethylenglycol oder die Mono-, Di-, Tri- und Tetraester von Pentaerythrit jeweils mit linearen gesättigten $C_{12}$ - $C_{30}$-Carbonsäuren, welche hydroxyliert sein können, insbesondere diejenigen mit Palmitin- und Stearinsäure, die Sorbitanmono-, -di- oder -triester von linearen gesättigten $C_{12}$ - $C_{30}$-Carbonsäuren, welche hydroxyliert sein können, insbesondere diejenigen von Myristinsäure, Palmitinsäure, Stearinsäure oder von Mischungen dieser Fettsäuren und die Methylglucosemono- und -diester von linearen gesättigten $C_{12}$ - $C_{30}$-Carbonsäuren, welche hydroxyliert sein können.

Im Rahmen der vorliegenden Erfindung kann es vorgesehen sein, dass die erfindungsgemäßen kosmetischen Mittel mindestens einen Polyolpartialester, ausgewählt aus Glycerinmonostearat, Glycerinmonopalmitat, Glycerindistearat, Glycerindipalmitat, Ethylenglycolmonostearat, Ethylenglycolmonopalmitat, Ethylenglycoldistearat, Ethylenglycoldipalmitat, sowie Mischungen hiervon, insbesondere Mischungen aus Glycerinmonostearat, Glycerinmonopalmitat, Glycerindistearat und Glycerindipalmitat in einer Gesamtmenge von 0,5 bis 10 Gew.-%, insbesondere von 3,0 bis 8,0 Gew.-%, bezogen auf das Gesamtgewicht des kosmetischen Mittels, enthalten.

Der Einsatz der zuvor angeführten Alkohole, Partialester und Polypartialester in den erfindungsgemäßen kosmetischen Mitteln kann insbesondere dann bevorzugt sein, wenn die erfindungsgemäßen kosmetischen Mittel in Form einer Öl-in-Wasser-Emulsion vorliegen.

Weiterhin kann es erfindungsgemäß vorgesehen sein, dass die kosmetischen Mittel gemäß der Erfindung mindestens ein Tensid enthalten. Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Gemäß einer bevorzugten Ausführungsform der vorliegenden Erfindung enthalten die erfindungsgemäßen kosmetischen Mittel mindestens ein amphoteres Tensid in einer Gesamtmenge von 0,1 bis 5,0 Gew.-%, insbesondere von 0,2 bis 2,0 Gew.-%, bezogen auf das Gesamtgewicht des kosmetischen Mittels. Als amphotere bzw. zwitterionische Tenside werden solche oberflächenaktiven Verbindungen bezeichnet, welche mindestens eine quartäre Ammoniumgruppe und mindestens eine - $COO^{(-)}$- oder -$SO3^{(-)}$-Gruppe aufweisen.

Als amphotere Tenside sind im Rahmen der vorliegenden Erfindung die nachfolgend genannten Verbindungen besonders bevorzugt:

- Alkylbetaine mit 8 bis 20 Kohlenstoffatomen in der Alkylgruppe,
- Amidopropylbetaine mit 8 bis 20 Kohlenstoffatomen in der Acylgruppe,
- Sulfobetaine mit 8 bis 20 Kohlenstoffatomen in der Acylgruppe, und
- Amphoacetate oder Amphodiacetate mit 8 bis 20 Kohlenstoffatomen in der Acylgruppe.

In einer besonders bevorzugten Ausführungsform enthalten die erfindungsgemäßen kosmetischen Mittel als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das Gesamtgewicht des kosmetischen Mittels.

Weiterhin kann es vorgesehen sein, dass die erfindungsgemäßen kosmetischen Mittel mindestens ein ethoxyliertes nichtionisches Tensid in einer Gesamtmenge von 0,5 bis 6,0 Gew.-%, insbesondere von 1,0 bis 4,0 Gew.-%, bezogen auf das Gesamtgewicht des kosmetischen Mittels, enthalten. Dabei hat es sich als besonders vorteilhaft erwiesen, wenn das ethoxylierte nichtionische Tensid einen HLB-Wert oberhalb von 10, vorzugsweise oberhalb von 13 aufweist. Hierzu ist es erforderlich, dass das nichtionische Tensid einen ausreichend hohen Ethoxylierungsgrad besitzt. In diesem Zusammenhang enthält das erfindungsgemäße kosmetische Mittel daher als ethoxyliertes nichtionisches Tensid mindestens ein ethoxyliertes Tensid mit mindestens 12 Ethylenoxideinheiten. Neben den entsprechend ethoxylierten Fettalkoholen, insbesondere Laurylalkohol, Myristylalkohol, Cetylalkohol, Stearylalkohol, Arachylalkohol und Behenylalkohol, sind erfindungsgemäß insbesondere die Anlagerungsprodukte von 20 bis 60 Mol Ethylenoxid an Rizinusöl und gehärtetes Rizinusöl besonders geeignet. Das mindestens eine ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30.

Kosmetische Mittel im Rahmen der vorliegenden Erfindung weisen in der Regel einen basischen pH-Wert, insbesondere zwischen pH 8,0 und pH 12, auf. Diese pH-Werte sind erforderlich, um eine Öffnung der äußeren Schuppenschicht (Cuticula) zu gewährleisten und eine Penetration der Oxidationsfarbstoffvorprodukte und/oder des Oxidationsmittels in das Haar zu ermöglichen.

Die Einstellung des zuvor genannten pH-Wertes kann bevorzugt unter Verwendung eines Alkalisierungsmittels erfolgen. Im Rahmen der vorliegenden Erfindung ist das Alkalisierungsmittel ausgewählt aus der Gruppe von (i) anorganischen Alkalisierungsmitteln; (ii) organischen Alkalisierungsmitteln; sowie (iii) deren Mischungen, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels, enthalten.

Bevorzugte anorganische Alkalisierungsmittel sind ausgewählt aus der Gruppe, welche gebildet wird aus Ammoniak bzw. Ammoniumhydroxid, also wässrigen Lösungen von Ammoniak, Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Ammoniak bzw. Ammoniumhydroxid ist ein besonders bevorzugtes Alkalisierungsmittel. Besonders bevorzugt ist Ammoniak in einer Gesamtmenge von 0,1 bis 20 Gew.-%, vorzugsweise von 0,5 bis 10 Gew.-%, insbesondere von 1,0 bis 7,0 Gew.-%, bezogen auf das Gesamtgewicht des kosmetischen Mittels, enthalten.

Bevorzugte organische Alkalisierungsmittel sind ausgewählt aus mindestens einem Alkanolamin. Erfindungsgemäß bevorzugte Alkanolamine sind ausgewählt aus Alkanolaminen aus primären, sekundären oder tertiären Aminen mit einem $C_2$-$C_6$-Alkylgrundkörper, welcher mindestens eine Hydroxylgruppe trägt. Besonders bevorzugte Alkanolamine werden aus der Gruppe ausgewählt, welche gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine werden ausgewählt aus der Gruppe von 2-Aminoethan-1-ol (Monoethanolamin), 2-Amino-2-methyl-propan-1-ol, 2-Amino-2-methyl-propan-1,3-diol und Triethanolamin. Besonders bevorzugte erfindungsgemäße kosmetische Mittel enthalten eine Mischung aus Monoethanolamin und 2-Amino-2-methylpropan-1ol. Bevorzugt ist das mindestens eine Alkanolamin in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels, enthalten.

Weitere erfindungsgemäß bevorzugte organische Alkalisierungsmittel sind ausgewählt aus basischen Aminosäuren, besonders bevorzugt ausgewählt aus der Gruppe, welche gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte erfindungsgemäße kosmetische Mittel enthalten mindestens ein von Alkanolaminen und Ammoniak verschiedenes Alkalisierungsmittel in einer Gesamtmenge von 0,05 bis 5,0 Gew.-%, insbesondere von 0,5 bis 3,0 Gew.-%, bezogen auf das Gesamtgewicht des kosmetischen Mittels.

Erfindungsgemäß bevorzugt ist das Alkalisierungsmittel ausgewählt aus der Gruppe von Natriumhydroxid, Kaliumhydroxid, Ammoniak, Monoethanolamin und 2-Amino-2-methylpropan, vorzugsweise Monoethanolamin, und ist in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels, enthalten.

In einer besonders bevorzugten Ausführungsform enthalten die erfindungsgemäßen kosmetischen Mittel als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels.

Bevorzugt beträgt der pH-Wert der erfindungsgemäßen kosmetischen Mittel, gemessen bei 22°C, 8 bis 13, vorzugsweise 9,5 bis 12, bevorzugt 10 bis 11,5, insbesondere 10,5 bis 11.

Im Rahmen der vorliegenden Erfindung kann es weiterhin bevorzugt sein, wenn die erfindungsgemäßen kosmetischen Mittel mindestens ein Öl, ausgewählt aus der Gruppe von Sonnenblumenöl, Maisöl, Sojaöl, Kürbiskernöl, Traubenkernöl, Sesamöl, Haselnussöl, Aprikosenkernöl, Macadamianussöl, Araraöl, Rizinusöl, Avocadoöl sowie deren Mischungen, in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels, enthalten. Durch den Einsatz mindestens

eines zuvor genannten Öls kann der Pflegeeffekt der aminierten Siliconpolymere weitergehend gesteigert werden.

Besonders bevorzugt enthalten die erfindungsgemäßen kosmetischen Mittel Traubenkernöl in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels.

Gemäß einer besonders bevorzugten Ausführungsform der vorliegenden Erfindung enthalten die als Öl-in-Wasser-Emulsion vorliegenden erfindungsgemäßen kosmetischen Mittel - bezogen auf das Gesamtgewicht der kosmetischen Mittel -

- Octyldodecanol in einer Gesamtmenge von 2,0 bis 20 Gew.-%, insbesondere von 5,0 bis 12 Gew.-%, weiterhin
- Mischungen aus Glycerinmonostearat, Glycerinmonopalmitat, Glycerindistearat und Glycerindipalmitat in einer Gesamtmenge von 0,5 bis 10 Gew.-%, bevorzugt 3,0 bis 8,0 Gew.-%, weiterhin
- mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, weiterhin
- eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, weiterhin
- Traubenkernöl in einer Gesamtmenge von 0,1 bis 10 Gew.-%, bevorzugt von 0,2 bis 5,0 Gew.-%, insbesondere von 0,5 bis 2,0 Gew.-%.

Oxidative Aufhellmittel können auch direkt vor der Anwendung aus zwei oder mehreren getrennt verpackten Zusammensetzungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme ist insbesondere dort bevorzugt, wo Inkompatibilitäten der Inhaltsstoffe zu erwarten oder zu befürchten sind. Das oxidative Aufhellmittel wird in diesen Fällen vom Verbraucher direkt vor der Anwendung durch Vermischen der Komponenten hergestellt. Im Rahmen der vorliegenden Erfindung ist dieses Vorgehen bei oxidativen Aufhellmitteln, bei welchen das erfindungsgemäße kosmetische Mittel zunächst getrennt von einer Oxidationsmittelzubereitung, enthaltend mindestens ein Oxidationsmittel, vorliegt, besonders bevorzugt.

Ein weiterer Gegenstand der vorliegenden Erfindung ist daher eine Verpackungseinheit (Kit-of-Parts), umfassend - getrennt voneinander konfektioniert -

a) mindestens einen Container (C1), enthaltend ein erfindungsgemäßes kosmetisches Mittel, und

b) mindestens einen Container (C2), enthaltend eine Oxidationsmittelzubereitung, welche in einem kosmetisch verträglichen Träger mindestens ein Oxidationsmittel enthält.

Unter dem Begriff "Container" wird im Rahmen der vorliegenden Erfindung eine Umhüllung verstanden, welche in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich dabei jedoch um Umhüllungen aus Glas oder Kunststoff.

Die Oxidationsmittel im Rahmen der vorliegenden Erfindung sind von Luftsauerstoff verschieden. Als Oxidationsmittel können Wasserstoffperoxid sowie dessen feste Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Erfindungsgemäß bevorzugt ist das Oxidationsmittel daher ausgewählt aus der Gruppe von Persulfaten, Chloriten, Wasserstoffperoxid und Anlagerungsprodukten von Wasserstoffperoxid an Harnstoff, Melamin sowie Natriumborat, insbesondere Wasserstoffperoxid.

Ein besonders bevorzugte Ausführungsform der vorliegenden Erfindung ist somit dadurch gekennzeichnet, dass als Oxidationsmittel Wasserstoffperoxid in einer Gesamtmenge von 0,5 bis 20 Gew.-%, vorzugsweise von 2,0 bis 15 Gew.-%, bevorzugt von 3,0 bis 12 Gew.-%, besonders bevorzugt von 4,0 bis 10 Gew.-%, insbesondere von 5,5 bis 9,0 Gew.-%, bezogen auf das Gesamtgewicht der Oxidationsmittelzubereitung, enthalten ist. Die Berechnung der Gesamtmenge bezieht sich hierbei auf 100 %-iges $H_2O_2$.

Die Oxidationsmittelzubereitungen können weiterhin Wasser in einer Gesamtmenge von 40 bis 98 Gew.-%, insbesondere von 65 bis 85 Gew.-%, bezogen auf das Gesamtgewicht der Oxidationsmittelzubereitung, enthalten.

Gemäß einer bevorzugten Ausführungsform der vorliegenden Erfindung enthalten die Oxidationsmittelzubereitungen weiterhin mindestens ein lineares gesättigtes Alkanol mit 12 bis 30 Kohlenstoffatomen, insbesondere mit 16 bis 22 Kohlenstoffatomen, in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung. Bevorzugt sind insbesondere Cetylalkohol, Stearylalkohol, Arachidylalkohol, Behenylalkohol und Lanolinalkohol oder Gemische dieser

Alkohole, wie sie bei der großtechnischen Hydrierung von pflanzlichen und tierischen Fettsäuren erhältlich sind, sowie Mischungen dieser Alkanole. Besonders bevorzugt ist die Mischung Cetearylalkohol.

In einer weiteren bevorzugten Ausführungsform der vorliegenden Erfindung enthalten die Oxidationsmittelzubereitungen mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1 bis 4,0 Gew.-%, bezogen auf das Gesamtgewicht der Oxidationsmittelzubereitung.

Im Rahmen der vorliegenden Erfindung kann es darüber hinaus auch vorgesehen sein, dass die Oxidationsmittelzubereitungen mindestens einen Ester aus einer Carbonsäure mit 10 bis 20 Kohlenstoffatomen und einem linearen oder verzweigten Alkohol mit 1 bis 5 Kohlenstoffatomen, insbesondere Isopropylmyristat, in einer Gesamtmenge von 3,0 bis 25 Gew.-%, vorzugsweise von 5,0 bis 20 Gew.-%, insbesondere von 8,0 bis 15 Gew.-%, bezogen auf das Gesamtgewicht der Oxidationsmittelzubereitung, enthalten.

Gemäß einer besonders bevorzugten Ausführungsform der vorliegenden Erfindung enthalten die Oxidationsmittelzubereitungen - bezogen auf das Gesamtgewicht der Oxidationsmittelzubereitungen -

- mindestens ein lineares gesättigtes Alkanol mit 12 bis 30 Kohlenstoffatomen in einer Gesamtmenge von 0,1 bis 10 Gew.-%, vorzugsweise von 0,5 bis 5,0 Gew.-%, insbesondere von 1,0 bis 4,0 Gew.-%, weiterhin
- mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1,0 bis 4,0 Gew.-%, sowie
- mindestens einen Ester aus einer Carbonsäure mit 10 bis 20 Kohlenstoffatomen und einem linearen oder verzweigten Alkohol mit 1 bis 5 Kohlenstoffatomen, bevorzugt Isopropylmyristat, in einer Gesamtmenge von 3,0 bis 25 Gew.-%, vorzugsweise von 5,0 bis 20 Gew.-%, insbesondere von 8,0 bis 15 Gew.-%.

Die erfindungsgemäßen Oxidationsmittelzubereitungen enthalten weiterhin mindestens eine Säure. Bevorzugte Säuren sind ausgewählt aus Dipicolinsäure, Genusssäuren, wie beispielsweise Zitronensäure, Essigsäure, Apfelsäure, Milchsäure und Weinsäure, verdünnten Mineralsäuren, wie Salzsäure, Phosphorsäure, Pyrophosphorsäure und Schwefelsäure, sowie Mischungen hiervon. Die Oxidationsmittelzubereitungen weisen bevorzugt einen pH-Wert im Bereich von 2 bis 5, insbesondere von 3 bis 4, auf.

Zur Herstellung von oxidativen Aufhellmittel aus der erfindungsgemäßen Verpackungseinheit (Kit-of-parts) wird das erfindungsgemäße kosmetische Mittel in dem Container C1 mit der Oxidationsmittelzubereitung in dem Container C2 oder *vice versa* vermischt.

Weiterhin kann es erfindungsgemäß besonders vorteilhaft sein, wenn die Verpackungseinheit mindestens ein weiteres Haarbehandlungsmittel, insbesondere eine Konditioniermittelzubereitung, in einem zusätzlichen Container enthält. Diese Konditioniermittelzubereitung enthält vorteilhafterweise mindestens ein Konditioniermittel, ausgewählt aus der Gruppe von kationischen Polymeren, Siliconderivaten und Ölen. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, welche das Abteilen von Faserbündeln bzw. Strähnchen aus der Gesamtmenge der Fasern erlaubt und vereinfacht.

Bezüglich des erfindungsgemäßen kosmetischen Mittels in dem Container C1 und der Oxidationsmittelzubereitung in dem Container C2 gilt mutatis mutandis das zu den erfindungsgemäßen kosmetischen Mitteln Gesagte.

Ein weiterer Gegenstand der vorliegenden Erfindung ist ein Verfahren zur Färbung, insbesondere Aufhellung, keratinischer Fasern, wobei das Verfahren die folgenden Verfahrensschritte umfasst:

a) Bereitstellen eines erfindungsgemäßen kosmetischen Mittels (M1),
b) Bereitstellen einer Oxidationsmittelzubereitung (M2), enthaltend in einem kosmetisch verträglichen Träger mindestens ein Oxidationsmittel,
c) Vermischen des kosmetischen Mittels (M1) mit der Oxidationsmittelzubereitung (M2),
d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 45 °C auf den keratinischen Fasern,
e) Spülen der keratinischen Fasern mit Wasser und/oder einer reinigenden Zusammensetzung für 1 bis 5 Minuten,

und

f) gegebenenfalls Applizieren eines Nachbehandlungsmittels auf die keratinischen Fasern und Ausspülen nach einer Zeit von 1 bis 10 Minuten.

Das erfindungsgemäße Verfahren zur Färbung, insbesondere Aufhellung, keratinischer Fasern unter Verwendung eines speziellen aminierten Siliconpolymers resultiert in einer verbesserten Pflege gefärbter keratinischer Fasern bei gleichzeitig verbesserter Aufhellleistung.

Unter Raumtemperatur ist dabei im Rahmen der vorliegenden Erfindung die Umgebungstemperatur zu verstehen, welche ohne Einwirkung von externer Wärme vorherrscht und bevorzugt von 10 bis 39 °C beträgt. Die Wirkung der Aufhellzubereitung kann durch externe Wärmezufuhr, beispielsweise mittels einer Wärmehaube, verstärkt werden. Die bevorzugte Einwirkdauer der Aufhellzubereitung auf die keratinischen Fasern beträgt 10 bis 60 min, bevorzugt 20 bis 45 min. Nach Beendigung der Einwirkdauer wird das verbliebene Aufhellmittel aus den keratinischen Fasern mit Hilfe einer Reinigungszubereitung, welche bevorzugt mindestens ein kationisches und/oder anionisches und/oder nichtionisches Tensid enthält, und/oder Wasser ausgewaschen. Gegebenenfalls wird der Vorgang mit einem weiteren Mittel wiederholt. Nach dem Auswaschen werden die keratinischen Fasern gegebenenfalls mit einem Nachbehandlungsmittel, beispielsweise einem Konditioniermittel, gespült und mit einem Handtuch oder einem Heißluftgebläse getrocknet. Die Auftragung der Aufhellzusammensetzung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbemittel mit einer Applikationshilfe auf die keratinischen Fasern aufzutragen.

Besonders bevorzugte erfindungsgemäße Verfahren sind dadurch gekennzeichnet, dass die Verfahren in einer verbesserten Pflege der keratinischen Fasern bei gleichzeitig verbesserter Aufhellleistung resultieren. Durch die Verwendung mindestens eines speziellen aminierten Siliconpolymers ist die aus dem erfindungsgemäßen Verfahren resultierende Pflege und Aufhellleistung größer als die Pflege und Aufhellleistung, welche in Abwesenheit des erfindungsgemäß eingesetzten aminierten Siliconpolymers b) erreicht werden kann.

Bezüglich des erfindungsgemäßen kosmetischen Mittels M1, der Oxidationsmittelzubereitung M2 sowie weiterer bevorzugter Ausführungsformen des erfindungsgemäßen Verfahrens gilt mutatis mutandis das zu den erfindungsgemäßen kosmetischen Mitteln sowie zu der erfindungsgemäßen Verpackungseinheit Gesagte.

Die nachfolgenden Beispiele sind nicht erfindungsgemäss.

Beispiele:

1. Rezepturen

Zusammensetzungen der eingesetzten kosmetischen Mittel (Öl-in-Wasser-Emulsionen, alle Mengen in Gew.-%). Das in den nachfolgenden Formulierungen verwendete aminierte Siliconpolymer ist bevorzugt ein Siliconpolymer der Formel (III) mit n = 2 oder 3 und einem mittleren Molekulargewicht $M_w$ von 1.000 bis 200.000 Da.

| Rohstoff | V1 | E1* | E2* |
|---|---|---|---|
| Xanthan Gum | 0,05 | 0,05 | 0,05 |
| 2-Octyldodecanol | 2,3 | 2,3 | 2,3 |
| Lanette N [a)] | 14 | 14 | 14 |
| Cetearyl Alkohol | 3,9 | 3,9 | 3,9 |
| Glycerinmonostearat | 6,0 | 6,0 | 6,0 |
| Glycerol 99,5 % | 2,0 | 2,0 | 2,0 |
| Kokosamidopropylbeatin, 40%ig | 2,0 | 2,0 | 2,0 |
| Monoethanolamin | 6,1 | 6,1 | 6,1 |
| 2-Amino-2-methylpropanol | 0,1 | 0,1 | 0,1 |
| Natriumsulfit, wasserfrei | 0,1 | 0,1 | 0,1 |
| Caramelsirup, 75%ig | 0,1 | 0,1 | 0,1 |
| Traubenkernöl | 1,0 | 1,0 | 1,0 |

(fortgesetzt)

| Rohstoff | V1 | E1* | E2* |
|---|---|---|---|
| p-Toluylendiaminsulfat | 0,09 | 0,09 | 0,09 |
| 1,3-Benzoldiol | 0,02 | 0,02 | 0,02 |
| 3-Aminophenol | 0,003 | 0,003 | 0,003 |
| 4-Chlorresorcin | 0,03 | 0,03 | 0,03 |
| Aminiertes Siliconpolymer** | - | 0,97 | 1,9 |
| Wasser, vollentsalzt | ad 100,00 | ad 100,00 | ad 100,00 |
| * erfindungsgemäß<br>** Aktivsubstanz<br>[a)] INCI-Bezeichnung: Cetearyl alcohol, Sodium cetearyl sulfate (BASF) | | | |

Die Fettbasis wurde jeweils zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V1 handelt es sich um eine Rezeptur ohne aminiertes Siliconpolymer. Die Rezepturen E1 und E2 sind Rezepturen mit linearem aminiertem Siliconpolymer. Oxidationsmittelzubereitung O1 (alle Mengen in Gew.-%)

| Rohstoff | O1 |
|---|---|
| Dinatriumpyrophosphat | 0,10 |
| Dipicolinsäure | 0,10 |
| Kaliumhydroxid 50% | 0,23 |
| 1-Hydroxyethan-1,1-diphosphonsäure 60% | 0,25 |
| Emulgade F [b)] | 4,0 |
| Cetearyl Alcohol | 0,5 |
| Ceteareth-20 | 0,5 |
| Bienenwachs | 0,3 |
| Isopropylmyristat | 10 |
| Wasserstoffperoxid 50 % | 23 |
| Wasser vollentsalzt | ad 100 |
| [b)] INCI-Bezeichnung: Cetearyl alcohol, PEG-40 Castor oil, Sodium cetearyl sulfat (BASF) | |

2. Verbesserte Pflege durch Zusatz mindestens eines speziellen aminierten Siliconpolymers

Zur Herstellung der oxidativen Aufhellmittel zur Bestimmung der Pflege wurden die kosmetischen Mittel V1, E1 und E2 jeweils im Gewichtsverhältnis 1:2 mit der obigen Oxidationsmittelzubereitung O1 vermischt.

12 Strähnen von natürlich hellbraunem europäischen Haar (IHIP (New York), lot # 03/2012, N121, lenght 15 cm, weight 1 g) wurden mit einer wässrigen Natrium-Laurylethersulfat-Lösung (3 % Aktivsubstanzgehalt in der Lösung) gewaschen. Die Strähnen wurden an der Luft getrocknet und für 24 h bei 25°C und 25% relativer Luftfeuchtigkeit gelagert. Nach Einweichen dieser Strähnen für 5 Minuten in Wasser wurde deren Nasskämmbarkeit bestimmt (Referenzwert).

Für die Färbungen wurden jeweils 12 Strähnen von natürlich europäischen Haar (IHIP (New York), lot # 03/2012, N121, lenght 15 cm, weight 1 g) je oxidativem Färbemittel verwendet. Hierfür wurden jeweils 4 g der zuvor hergestellten oxidativen Aufhellmittel pro 1 g Haarsträhne appliziert. Nachdem die Strähnen für 30 min bei 32 °C gefärbt wurden, wurden sie für 2 min mit Wasser gespült und an der Luft getrocknet.

Die Messung der Nasskämmbarkeit wurde wie folgt durchgeführt:
Vor der Messung wurde jede Strähne unter Kämmen mit einem harten Gummikamm mit feinen Zähnen (Firma Hercules Sägemann, Hamburg Germany) für 2 Sekunden mit Wasser befeuchtet. Nachdem 3 Kämmvorgänge durchgeführt

wurden, wird die Kämmkraft während weiteren 10 Kämmvorgängen gemessen, wobei die jeweilige Haarsträhne während des Kämmvorgangs langsam rotiert. Die erhaltenen Messwerte werden unter Verwendung der folgenden in der Software Statistica 10.0 (StatSoft Inc., USA) eingebetteten statistischen Tests verglichen:

- Shapiro-Wilks Test (Test für Normalverteilung)
- Ausreißertest nach Grubbs
- Bartlett-Test (Test auf Homoskedastizität von Varianzen)
- Univarianter Signifikanztest
- Newman-Keuls Test (Bestimmung von signifikanten Unterschieden)
- Unequal N HSD Test (Test auf Mehrfachvergleiche).

Die Änderung der Kämmkraft dK in Prozent kann mit Hilfe der Formel $dK = [(K_0-K_i)/K_0]*100$ berechnet werden. $K_0$ ist hierbei der Mittelwert der Kämmkraft für die ungefärbten Haarsträhnen und $K_i$ der Mittelwert für die mit dem jeweiligen oxidativen Aufhellmitteln behandelten Haarsträhnen.

Die Pflege der Haarsträhnen ist umso höher, je geringer die aufgewendete Kämmkraft und damit je höher die Änderung der Kämmkraft ist. In Tabelle 2 sind die dK-Werte für die Ausfärbungen unter Verwendung der kosmetischen Mittel V1, E1 und E2 dargestellt. Die Ausfärbungen mit den erfindungsgemäßen kosmetischen Mitteln E1 und E2, welche mindestens ein spezielles aminiertes Siliconpolymer in einer Gesamtmenge von 0,97 Gew.-% bzw. 1,9 Gew.-% enthalten, weisen im Vergleich zu den Ausfärbungen ohne aminiertes Siliconpolymer (V1) eine höhere Änderung der Kämmkraft und somit eine erhöhte Pflege auf.

| Oxidatives Färbemittel | dK [%] |
|---|---|
| V1 + O1 (1:2) | 23 |
| E1 + O1 (1:2) | 42 |
| E2 + O1 (1:2) | 40 |

3. Verbesserte Aufhellleistung durch Zusatz mindestens eines speziellen aminierten Siliconpolymers

Für den Aufhellprozess wurde auf Strähnen von dunkelblonden, hellbraunen und dunkelbraunen Haar (Codes: Kerling 6/0, Fischbach & Miller 6923) von ca. 0,7 g Gewicht die 4-fache Menge der unter Punkt 2. Hergestellten oxidativen Aufhellmittel appliziert. Nachdem die Strähnen für 45 min bei 32 °C blondiert wurden, wurden sie mit einem handelsüblichen Shampoo gewaschen und mit einem Föhn getrocknet.

Alle Strähnen wurden mit einem Farbmessgerät der Firma Datacolor, Typ Spectraflash 450, vermessen. Der für die Beurteilung der Aufhellleistung herangezogene Wert dL ergibt sich aus den an der jeweiligen Strähne gemessenen L*a*b-Farbmesswerten wie folgt:

$$dL = L_i - L_0$$

$L_0$ ist hierbei jeweils der Mittelwert der aus den 12 Messungen ermittelten Farbmesswerte der unbehandelten Haarsträhnen, während $L_i$ jeweils den Mittelwert der Farbmesswerte nach der Aufhellung der Haarsträhnen mit dem jeweiligen oxidativen Aufhellmittel darstellt.

Je höher der dL-Wert ist, desto höher ist die Aufhellleistung des entsprechenden kosmetischen Mittels. In der nachfolgenden Tabelle sind die dL-Werte für die Ausfärbungen unter Verwendung der kosmetischen Mittel V1, E1 und E2 dargestellt. Die Ausfärbungen mit den erfindungsgemäßen kosmetischen Mitteln E1 und E2, welche mindestens ein aminiertes Siliconpolymer in einer Gesamtmenge von 0,97 Gew.-% bzw. 1,9 Gew.-% enthalten, weisen im Vergleich zu der Ausfärbung ohne aminiertes Siliconpolymer (V1) eine verbesserte Aufhellleistung auf.

| Oxidatives Aufhellmittel | dL |
|---|---|
| V1 + O1 (1:2) | 5,87 |
| E1 + O1 (1:2) | 7,27 |
| E2 + O1 (1:2) | 6,27 |

**Patentansprüche**

**1.** Kosmetisches Mittel zur Farbveränderung, insbesondere Aufhellung, keratinischer Fasern, umfassend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,
b) mindestens ein aminiertes Siliconpolymer, enthaltend mindestens eine Struktureinheit der Formel (I) und mindestens eine Struktureinheit der Formel (II)

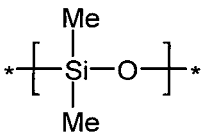 (I) 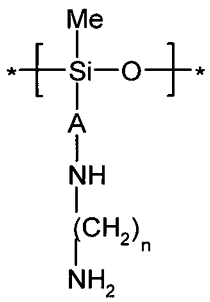 (II),

worin

A für eine lineare oder verzweigte $C_4$-$C_8$-Alkylgruppe steht, und
n für ganze Zahlen von 1 bis 4 steht,

wobei

- das kosmetische Mittel keine Säure(n), ausgewählt aus der Gruppe der Carbonsäuren mit 8 bis 30 Kohlenstoffatomen, der Ethercarbonsäuren mit 8 bis 30 Kohlenstoffatomen, der Etherphosphorsäuren mit 8 bis 30 Kohlenstoffatomen, der Phosphorsäuren mit 8 bis 30 Kohlenstoffatomen sowie deren Mischungen, enthält;
- das kosmetische Mittel mindestens ein aminiertes Siliconpolymer der Formel (III) enthält

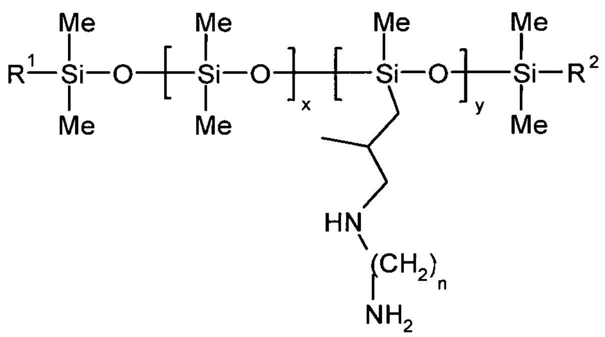 (III),

worin

$R^1$ und $R^2$, jeweils unabhängig voneinander, für eine Methylgruppe oder eine Hydroxylgruppe stehen,
x für ganze Zahlen von 49 bis 149, steht,
y für ganze Zahlen von 1 bis 10, steht, und
n für 2 oder 3, steht

- das kosmetische Mittel zusätzlich ein cyclisches aminiertes Siliconpolymer in einer Gesamtmenge von 0,015 bis 0,3 Gew.-%, bezogen auf das Gesamtgewicht des kosmetischen Mittels, enthält, wobei das cyclische aminierte Siliconpolymer die Formel (IV) aufweist

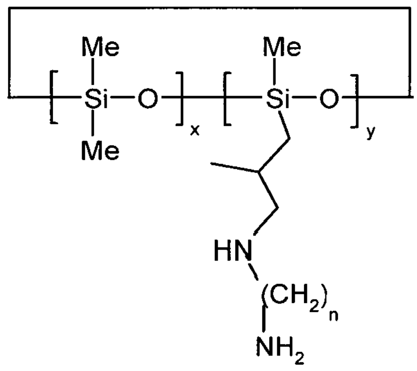

(IV),

worin

x für ganze Zahlen von 49 bis 149, steht, und
y für ganze Zahlen von 1 bis 10, steht.

2. Kosmetisches Mittel nach Anspruch 1, **dadurch gekennzeichnet, dass** das mindestens eine aminierte Siliconpolymer b) ein mittleres Molekulargewicht $M_w$ von 350 bis 350.000 Da, vorzugsweise von 500 bis 300.000 Da, bevorzugt von 700 bis 250.000 Da, insbesondere von 1.000 bis 200.000 Da, aufweist.

3. Kosmetisches Mittel nach einem der Ansprüche 1 oder 2, **dadurch gekennzeichnet, dass** das mindestens eine aminierte Siliconpolymer b) eine Aminzahl von 0,25 bis 5 meq/g, vorzugsweise von 0,3 bis 4,5 meq/g, bevorzugt von 0,4 bis 4,0 meq/g, weiter bevorzugt von 0,5 bis 3,0 meq/g, insbesondere von 0,5 bis 1,5 meq/g, aufweist.

4. Kosmetisches Mittel nach einem der vorangehenden Ansprüche, **dadurch gekennzeichnet, dass** das kosmetische Mittel das mindestens eine aminierte Siliconpolymer b) in einer Gesamtmenge von 0,0001 bis 15 Gew.-%, vorzugsweise von 0,0005 bis 10 Gew.-%, bevorzugt von 0,005 bis 5,0 Gew.-%, weiter bevorzugt von 0,01 bis 3,0 Gew.-%, insbesondere von 0,05 bis 1,0 Gew.-%, bezogen auf das Gesamtgewicht des kosmetischen Mittels, enthält.

5. Kosmetisches Mittel nach einem der vorangehenden Ansprüche, **dadurch gekennzeichnet, dass** das kosmetische Mittel zusätzlich Dimethylcyclosiloxan in einer Gesamtmenge von weniger als 1 Gew.-%, bezogen auf das Gesamtgewicht des kosmetischen Mittels, enthält, wobei das Dimethylcyclosiloxan die Formel (V) aufweist

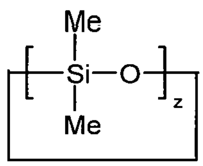

(V),

worin
z für 3, 4, 5 oder 6, steht.

6. Kosmetisches Mittel nach einem der vorangehenden Ansprüche, **dadurch gekennzeichnet, dass** das kosmetische Mittel zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

7. Kosmetisches Mittel nach Anspruch 6, **dadurch gekennzeichnet, dass** als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels, enthalten ist.

8. Verpackungseinheit (Kit-of-Parts), umfassend - getrennt voneinander konfektioniert -

a) mindestens einen Container (C1), enthaltend ein kosmetisches Mittel nach einem der Ansprüche 1 bis 7, und
b) mindestens einen Container (C2), enthaltend eine Oxidationsmittelzubereitung, welche in einem kosmetisch verträglichen Träger mindestens ein Oxidationsmittel enthält.

**9.** Verpackungseinheit (Kit-of-Parts), nach Anspruch 8, **dadurch gekennzeichnet, dass** das Oxidationsmittel Wasserstoffperoxid ist und in einer Gesamtmenge von 0,5 bis 20 Gew.-%, vorzugsweise von 2,0 bis 15 Gew.-%, bevorzugt von 3,0 bis 12 Gew.-%, besonders bevorzugt von 4,0 bis 10 Gew.-%, insbesondere von 5,5 bis 9,0 Gew.-%, bezogen auf das Gesamtgewicht der Oxidationsmittelzubereitung, enthalten ist.

**10.** Verfahren zum Färben, insbesondere Aufhellen, von keratinischen Fasern, wobei das Verfahren die folgenden Verfahrensschritte umfasst:

a) Bereitstellen eines kosmetischen Mittels (M1) nach einem der Ansprüche 1 bis 7,
b) Bereitstellen einer Oxidationsmittelzubereitung (M2), enthaltend in einem kosmetisch verträglichen Träger mindestens ein Oxidationsmittel,
c) Vermischen des kosmetischen Mittels (M1) mit der Oxidationsmittelzubereitung (M2),
d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 45 °C auf den keratinischen Fasern,
e) Spülen der keratinischen Fasern mit Wasser und/oder einer reinigenden Zusammensetzung für 1 bis 5 Minuten, und
f) gegebenenfalls Applizieren eines Nachbehandlungsmittels auf die keratinischen Fasern und Ausspülen nach einer Zeit von 1 bis 10 Minuten.

## Claims

**1.** A cosmetic agent for dyeing, in particular for lightening, keratin fibers, comprising, in a cosmetically acceptable carrier,

a) at least one compound selected from the group of oxidation dye precursors, substantive dyes and mixtures thereof,
b) at least one aminated silicone polymer containing at least one structural unit of formula (I) and at least one structural unit of formula (II),

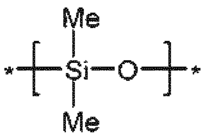
(I)

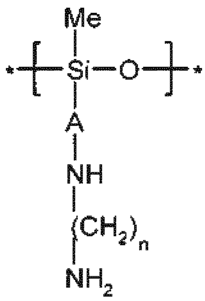
(II),

where

A represents a linear or branched $C_4$-$C_8$ alkyl group, and
n represents integers from 1 to 4,

wherein

- the cosmetic agent does not contain acid(s) selected from the group of carboxylic acids having 8 to 30 carbon atoms, ether carboxylic acids having 8 to 30 carbon atoms, ether phosphoric acids having 8 to 30 carbon atoms, phosphoric acids having 8 to 30 carbon atoms and mixtures thereof;
- the cosmetic agent contains at least one aminated silicone polymer of formula (III),

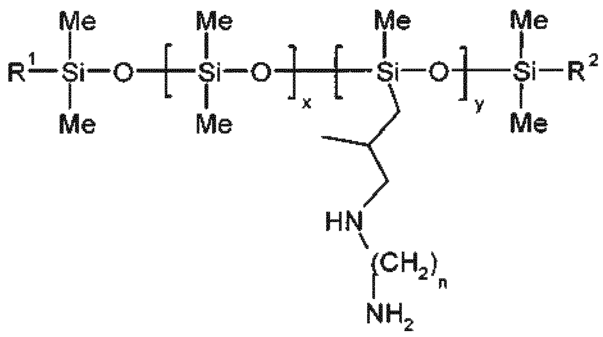

(III),

where

$R^1$ and $R^2$ each represent, independently of one another, a methyl group or a hydroxyl group,
x represents integers from 49 to 149,
y represents integers from 1 to 10, and
n represents 2 or 3,

- the cosmetic agent additionally contains a cyclic aminated silicone polymer in a total amount of from 0.015 to 0.3 wt.%, based on the total weight of the cosmetic agent, wherein the cyclic aminated silicone polymer has formula (IV),

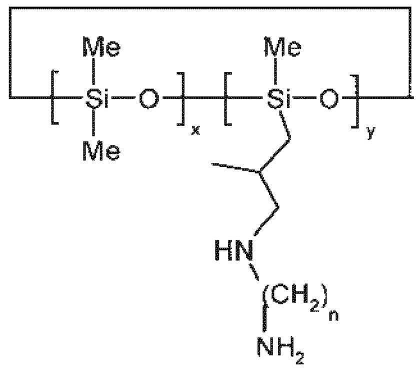

(IV),

where

x represents integers from 49 to 149, and
y represents integers from 1 to 10.

2. The cosmetic agent according to claim 1, **characterized in that** the at least one aminated silicone polymer b) has an average molecular weight $M_w$ of from 350 to 350,000 Da, preferably from 500 to 300,000 Da, more preferably from 700 to 250,000 Da, in particular from 1,000 to 200,000 Da.

3. The cosmetic agent according to claim 1 or 2, **characterized in that** the at least one aminated silicone polymer b) has an amine value of from 0.25 to 5 meq/g, preferably from 0.3 to 4.5 meq/g, more preferably from 0.4 to 4.0 meq/g, particularly preferably from 0.5 to 3.0 meq/g, in particular from 0.5 to 1.5 meq/g.

4. The cosmetic agent according to one of the preceding claims, **characterized in that** the cosmetic agent contains the at least one aminated silicone polymer b) in a total amount of from 0.0001 to 15 wt.%, preferably from 0.0005 to 10 wt.%, more preferably from 0.005 to 5.0 wt.%, even more preferably from 0.01 to 3.0 wt.%, in particular from 0.05 to 1.0 wt.%, based on the total weight of the cosmetic agent.

5. The cosmetic agent according to one of the preceding claims, **characterized in that** the cosmetic agent additionally contains dimethylcyclosiloxane in a total amount of less than 1 wt.%, based on the total weight of the cosmetic

agent, the dimethylcyclosiloxane having formula (V),

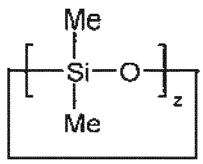

(V),

where
z represents 3, 4, 5 or 6.

6. The cosmetic agent according to one of the preceding claims, **characterized in that** the cosmetic agent additionally contains at least one further compound selected from the group of: (i) thickeners; (ii) linear or branched, saturated or unsaturated alcohols having 8 to 20 carbon atoms; (iii) surfactants, in particular amphoteric surfactants; (iv) alkalizing agents; (v) oils; and (vi) mixtures thereof.

7. The cosmetic agent according to claim 6, **characterized in that** a mixture of at least two different alkanolamines, in particular of monoethanolamine and 2-amino-2-methylpropan-1-ol, is contained as an alkalizing agent in a total amount of from 0.05 to 15 wt.%, preferably from 0.5 to 10 wt.%, in particular from 3.5 to 7.5 wt.%, based on the total weight of the cosmetic agent.

8. A packaging unit (kit-of-parts), comprising - packaged separately from one another -

a) at least one container (C1), containing a cosmetic agent according to one of claims 1 to 7, and
b) at least one container (C2), containing an oxidizing agent preparation which contains, in a cosmetically acceptable carrier, at least one oxidizing agent.

9. The packaging unit (kit-of-parts) according to claim 8, **characterized in that** the oxidizing agent is hydrogen peroxide and is contained in a total amount of from 0.5 to 20 wt.%, preferably from 2.0 to 15 wt.%, more preferably from 3.0 to 12 wt.%, particularly preferably from 4.0 to 10 wt.%, in particular from 5.5 to 9.0 wt.%, based on the total weight of the oxidizing agent preparation.

10. A method for dyeing, in particular lightening, keratin fibers, wherein the method comprises the following method steps:

a) providing a cosmetic agent (M1) according to one of claims 1 to 7,
b) providing an oxidizing agent preparation (M2) containing, in a cosmetically acceptable carrier, at least one oxidizing agent,
c) mixing the cosmetic agent (M1) with the oxidizing agent preparation (M2),
d) applying the mixture obtained in step c) to the keratin fibers and leaving said mixture on the keratin fibers for a time of from 10 to 60 minutes, preferably from 20 to 45 minutes, at room temperature and/or at at least 45 °C,
e) rinsing the keratin fibers with water and/or a cleaning composition for 1 to 5 minutes, and
f) optionally applying an after-treatment agent to the keratin fibers and rinsing said after-treatment agent after a time of from 1 to 10 minutes.

**Revendications**

1. Agent cosmétique destiné à modifier la couleur des fibres kératiniques, en particulier à les éclaircir, contenant, dans un support cosmétiquement acceptable,

a) au moins une liaison choisie dans le groupe des produits précurseurs de colorants par oxydation, des colorants montant directement sur la fibre ainsi que de leurs mélanges,
b) au moins un polymère de silicone aminé contenant au moins une unité structurelle de formule (I) et au moins une unité structurelle de formule (II)

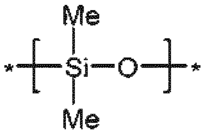 (I)

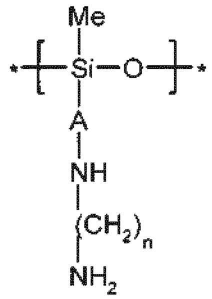 (II),

dans lesquelles

A représente un groupe alkyle en $C_4$-$C_8$ linéaire ou ramifié, et
n représente des nombres entiers compris entre 1 et 4,

dans lesquelles

- l'agent cosmétique ne contient pas d'acide(s) sélectionné(s) dans le groupe des acides carboxyliques ayant de 8 à 30 atomes de carbone, des acides carboxyliques d'éther ayant de 8 à 30 atomes de carbone, des acides phosphoriques d'éther ayant de 8 à 30 atomes de carbone, des acides phosphoriques ayant de 8 à 30 atomes de carbone ainsi que de leurs mélanges ;
- l'agent cosmétique contenant au moins un polymère de silicone aminé de formule (III)

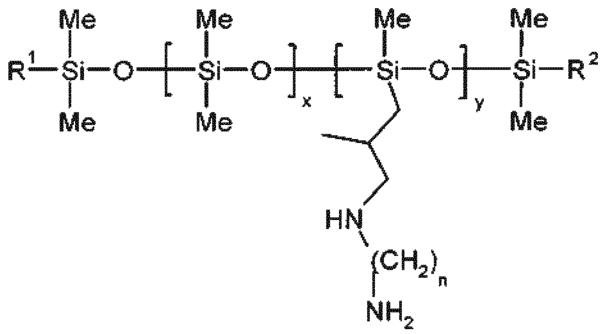 (III),

dans lesquelles

$R^1$ et $R^2$ représentent chacun indépendamment un groupe méthyle ou un groupe hydroxyle,
x représente des nombres entiers compris entre 49 et 149,
y représente des nombres entiers compris entre 1 et 10,
n représente 2 ou 3,

- l'agent cosmétique contient en outre un polymère de silicone aminé cyclique en une quantité totale de 0,015 à 0,3 % en poids par rapport au poids total de l'agent cosmétique, le polymère de silicone aminé cyclique présentant la formule (IV)

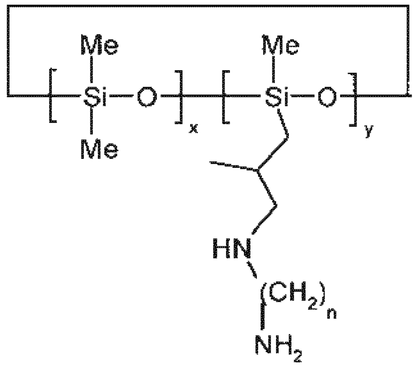

(IV),

dans lesquelles

x représente des nombres entiers compris entre 49 et 149, et
y représente des nombres entiers compris entre 1 et 10.

2. Agent cosmétique selon la revendication 1, **caractérisé en ce que** l'au moins un polymère de silicone aminé b) présente un poids moléculaire moyen $M_w$ de 350 à 350 000 Da, de préférence de 500 à 300 000 Da, de manière préférée de 700 à 250 000 Da, en particulier de 1 000 à 200 000 Da.

3. Agent cosmétique selon l'une quelconque des revendications 1 ou 2, **caractérisé en ce que** l'au moins un polymère de silicone aminé b) présente un indice d'amine de 0,25 à 5 meq/g, de préférence de 0,3 à 4,5 meq/g, de manière préférée de 0,4 à 4,0 meq/g, de manière davatage préférée de 0,5 à 3,0 meq/g, en particulier de 0,5 à 1,5 meq/g.

4. Agent cosmétique selon l'une quelconque des revendications précédentes, **caractérisé en ce que** l'agent cosmétique contient l'au moins un polymère de silicone aminé b) en une quantité totale de 0,0001 à 15 % en poids, de préférence de 0,0005 à 10 % en poids, de manière préférée de 0,005 à 5,0 % en poids, de manière davantage préférée de 0,01 à 3,0 % en poids, en particulier de 0,05 à 1,0 % en poids, par rapport au poids total de l'agent cosmétique.

5. Agent cosmétique selon l'une quelconque des revendications précédentes, **caractérisé en ce que** l'agent cosmétique contient en outre du diméthylcyclosiloxane en une quantité totale inférieure à 1 % en poids, par rapport au poids total de l'agent cosmétique, le diméthylcyclosiloxane présentant la formule (V)

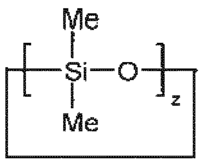

(V),

dans lesquelles
z représente 3, 4, 5 ou 6.

6. Agent cosmétique selon l'une quelconque des revendications précédentes, **caractérisé en ce que** l'agent cosmétique contient en outre au moins un autre composé choisi dans le groupe constitué (i) des épaississants ; (ii) des alcools linéaires ou ramifiés, saturés ou insaturés ayant de 8 à 20 atomes de carbone ; (iii) des tensioactifs, en particulier des tensioactifs amphotères ; (iv) des alcalinisants ; (v) des huiles ; ainsi que (vi) de leurs mélanges.

7. Agent cosmétique selon la revendication 6, **caractérisé en ce qu'**un mélange d'au moins deux alcanolamines différentes l'une de l'autre, en particulier de monoéthanolamine et de 2-amino-2-méthylpropan-1-ol, est présent comme alcalinisant, en une quantité totale de 0,05 à 15 % en poids, de préférence de 0,5 à 10 % en poids, en particulier de 3,5 à 7,5 % en poids, par rapport au poids total de l'agent cosmétique.

8. Unité de conditionnement (Kits-of-Parts) comprenant, fabriqués séparément les uns des autres,

a) au moins un récipient (C1) contenant un agent cosmétique selon l'une des revendications 1 à 7, et
b) au moins un contenant (C2), comprenant une préparation d'agent oxydant qui contient, dans un support cosmétique acceptable, au moins un agent oxydant.

**9.** Unité d'emballage (kit de pièces) selon la revendication 8, **caractérisée en ce que** l'agent oxydant est le peroxyde d'hydrogène et est présent en une quantité totale de 0,5 à 20 % en poids, de préférence de 2,0 à 15 % en poids, de préférence de 3,0 à 12 % en poids, de manière particulièrement préférée de 4,0 à 10 % en poids, en particulier de 5,5 à 9,0 % en poids, par rapport au poids total de la préparation d'agent oxydant.

**10.** Procédé de coloration, en particulier d'éclaircissement, de fibres kératiniques, le procédé comprenant les étapes de procédé suivantes :

a) la fourniture d'un agent cosmétique (M1) selon l'une quelconque des revendications 1 à 7,
b) la fourniture d'une préparation d'agent oxydant (M2) contenant, dans un support cosmétiquement acceptable, au moins un agent oxydant,
c) le mélange de l'agent cosmétique (M1) avec la préparation d'agent oxydant (M2),
d) l'application du mélange obtenu à l'étape c) sur les fibres kératiniques et le fait de laisser poser ce mélange sur une durée de 10 à 60 minutes, de préférence de 20 à 45 minutes, à température ambiante et/ou à au moins 45 °C sur les fibres kératiniques,
e) le rinçage des fibres kératiniques avec de l'eau et/ou une composition détergente pendant 1 à 5 minutes, et
f) éventuellement l'application d'un agent de post-traitement sur les fibres kératiniques et de rinçage après une durée de 1 à 10 minutes le cas échéant.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2003152534 A1
- WO 03009822 A2
- US 2003229947 A1
- WO 0247632 A2
- US 2007261178 A1
- EP 1464321 A1
- FR 2895242 A1
- WO 2012079915 A2


### In der Beschreibung aufgeführte Nicht-Patentliteratur


- **LIU X. M.** Comparative Studies of Poly(DimethylSiloxanes) Using Automated GPC-MALDI-TOF MS and On-Line GPC-ESI-TOF MS. *Am. Soc. Mass. Spectrom.,* 2003, vol. 14, 195-202